# EUROPEAN PATENT SPECIFICATION

(11) **EP 0 598 607 B1**
(45) Date of publication and mention of the grant of the patent: **16.02.2000**
(21) Application number: 93309173.8
(22) Date of filing: 17.11.1993
(51) Int. Cl.: A61B 17/28

(54) **Atraumatic endoscopic apparatus**
Atraumatisches endoskopisches Gerät
Instrument endoscopique atraumatique

(30) Priority: 18.11.1992 US 979496
(43) Date of publication of application: 25.05.1994
(73) Proprietor: ETHICON INC., Somerville New Jersey 08876 (US)
(72) Inventor: Adams, Ronald D., Cincinnati, Ohio 45215 (US); Thompson, Suzanne Elaine, West Chester, Ohio 45069 (US)
(74) Representative: Fisher, Adrian John

(56) References cited:
- EP-A- 0 542 437
- JP-A- 63 296 736
- US-A- 2 034 785
- US-A- 2 113 246
- US-A- 3 503 397
- US-A- 3 515 139
- US-A- 4 120 302

## Description

### Technical Field

The field of art to which this invention relates is surgical instrumentation, in particular endoscopic surgical instruments.

### Background of the Invention

The use of endoscopic surgical procedures has become increasingly common throughout the surgical community. There are many advantages associated with the use of endoscopic surgical techniques including decreased trauma, improved post-operative recuperation, decreased avenues for infection, and decreased post-operative hospital stays. The term endoscopic as used herein is meant to encompass all minimally invasive surgical techniques utilizing a scope including endoscopic, laparoscopic, thoracoscopic and arthroscopic.

In many endoscopic surgical techniques, it is necessary to enter a body cavity to obtain access to the target surgical site. This is conventionally done by using a trocar. A trocar typically consists of a trocar obturator having a sharp piercing point and a trocar cannula. The trocar obturator is concentrically housed within the trocar cannula during insertion through the musculature and fascia surrounding the body cavity. The trocar obturator is then removed from the trocar cannula after the trocar has been maneuvered into the body cavity, leaving the trocar cannula as a pathway into the body cavity, e.g., the abdomen.

Numerous surgical instruments have been developed and adapted for endoscopic surgical procedures. For example, there are stapling apparatuses, suture and cannula assemblies, electrocautery devices, tissue manipulating devices, tissue cutting devices, tissue ligating devices, and the like.

In most conventional, open surgical procedures, organs must typically be manually displaced by the surgeon to access a target surgical site. This must be done with minimal trauma to the organs. This task is facilitated during an open surgical procedure by the fact that the surgeon has sufficient tactile sensory input through a latex surgical glove to effectively prevent undue stress upon the organs when they are being displaced. In an endoscopic procedure it is also necessary for the surgeon to manipulate or move tissue including blood vessels internal organs such as the liver, the spleen, and the gall bladder in order to access a target surgical site. This is typically done with a variety of endoscopic tissue manipulators which have been specially developed for this task. However, there are certain deficiencies associated with these tissue manipulating devices. The tactile sensory input available in an open procedure is not available to the surgeon during a conventional endoscopic procedure since the endoscopic surgeon is manipulating organs and tissue with instruments. The surgeon, when manipulating organs with a manipulating instrument, has a loss of tactile sensory input. An additional complicating factor is that conventional endoscopes, having video output to video monitors, do not provide the surgeon with depth of field as the surgeon attempts to maneuver within the body cavity. Therefore, if the appropriate care is not taken by the surgeon, it is possible that organs and tissue, especially the capsular organs, will be traumatized or damaged by the endoscopic manipulating instruments as the surgeon attempts to maneuver in a three-dimensional space with a two-dimensional visualization system.

There is a need in this art for atraumatic endoscopic surgical manipulating instruments. Such instruments when used in a endoscopic surgical procedure would eliminate or minimize trauma to tissue and organs, in particular, capsular organs such as the liver, spleen and lungs.

Therefore, it is an object of the present invention to provide an atraumatic endoscopic surgical instrument for grasping and manipulating tissue and/or organs.

### Disclosure of the Invention

Accordingly, an atraumatic endoscopic apparatus for engaging mammalian tissue is disclosed. The endoscopic apparatus comprises a frame having a proximal end and a distal end. The frame has a passage therethrough. The endoscopic apparatus has a handle means at the proximal end of the frame for holding the apparatus. Jaw means are attached to the distal end of the frame for engaging or holding tissue or organs. The atraumatic endoscopic apparatus has actuating means for moving the jaw means between fully extended and fully closed positions. Atraumatic means are mounted to the jaw means effective to make the endoscopic apparatus substantially atraumatic when the jaw means are actuated by the actuating means.

The atraumatic means have the same durometer as a human finger.

The atraumatic means consists of a member having at least one tissue contact surface, preferably a substantially first planar tissue contact surface and a second proximal tissue contact surface angulated with respect to the first surface.

Another aspect of the present invention is a method of manipulating tissue or organs in an endoscopic procedure in a manner to effectively prevent or minimize trauma to the tissue or organs by using the above-described atraumatic endoscopic apparatus.

Yet another aspect of the present invention is an atraumatic means for mounting in an endoscopic apparatus for preventing or minimizing damage to tissue and organs.

Other features and advantages of the invention will become more apparent from the following description and accompanying drawings.

### Brief Description of the Drawings

FIG. 1 is perspective view of the atraumatic endoscopic apparatus of the present invention shown in an open position.
FIG. 2 is a partial perspective view of the distal end of the atraumatic endoscopic apparatus of the present invention shown in a closed position.
FIG. 3 is a partial cross-sectional view of the handle portion of the instrument as taken along View Line 3-3 of FIG. 1.
FIG. 4 is a partial cross-sectional view of the atraumatic endoscopic apparatus as taken along View Line 4-4 of FIG. 1 with the jaws in the open position.
FIG. 5 is an enlarged cross-sectional view of the atraumatic endoscopic apparatus as taken along View Line 5-5 of FIG. 2 with the jaws in a closed position.
FIG 6. is an exploded perspective view of the linkages used to articulate the jaws.
FIG. 7 is an exploded perspective view of the atraumatic means.
FIG. 8 is a perspective view of the atraumatic endoscopic apparatus of the present invention inserted through a trocar cannula into a mammalian body cavity.
FIG. 9 is a perspective view of an alternate embodiment of the atraumatic means shown with the jaw members in the open position.
FIG. 10 is a perspective view of the embodiment of FIG. 9 shown in a clamped position.
FIG. 11 is a perspective view of yet another embodiment of the atraumatic means which may be used with the atraumatic endoscopic apparatus of the present invention.

### Best Mode for Carrying Out the Invention

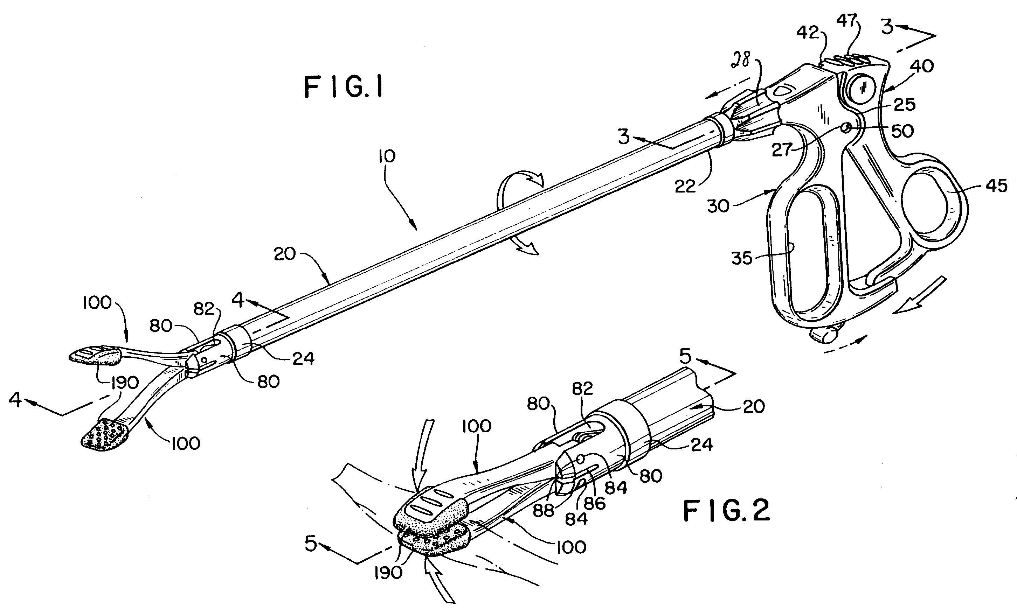

As can be seen in Figs. 1, 2, and 3, the atraumatic endoscopic apparatus 10 of the present invention has longitudinal frame 20. Longitudinal frame 20 has longitudinal passage 23 extending therethrough. The proximal end 22 of frame 20 is mounted to handle 30 which has finger grip 35 extending downwardly from the handle 30. Preferably, frame 20 is rotatably mounted to handle 30. Knob 28, mounted to proximal end 22 of frame 20, is used to rotate frame 20. Handle 30 has a pair of opposed mounting tabs 25 for mounting actuating lever 40. Mounting tabs 25 have holes 27 therethrough for receiving actuating lever pivot pin 50. Actuating lever 40 has pivot hole 49 for receiving pivot pin 50.

Actuating lever 40 is a substantially elongated member having thumb ring 45 at one end and head 42 at the opposite end. Head 42 has cavity 46 therein for receiving the proximal end 72 of actuating rod 70. Spherical cavity 41 is also contained in head 42 for mounting ball member 48. Pin hole 43 extends through ball member 48 for receiving actuator rod pivot pin 71. Ball member 48 has slot 48A for receiving distal end 72 and eye 75 of actuating rod 70. Although it is preferred that proximal end 72 be rotatably mounted in head 42 so that rod 70 can rotate when frame 20 is rotated, proximal end 72 may simply be pivotally mounted therein. Extending from the top of head 42 is optional thumb grip 47. Thumb grip 47 consists of a series of substantially parallel members disposed substantially perpendicular to the longitudinal axis of the apparatus 10. However, any conventional gripping means such as knurling and the like may be used. Actuating lever 40 is mounted to handle 30 at mounting tabs 25 by actuating lever pivot pin 50 which extends through pivot hole 49 and holes 27.

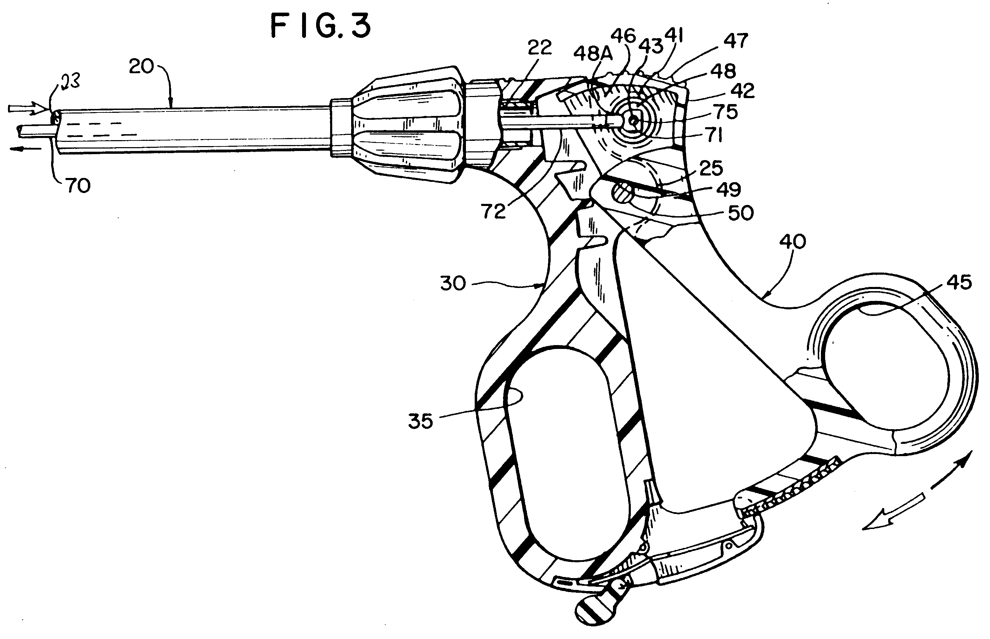

As can be seen with reference to FIGS. 3, 4, 5 and 6, actuating rod 70 is an elongated, substantially longitudinal member which is slideably mounted within frame 20 in passage 23. The actuating rod 70 has proximal end 72 and distal end 74. Proximal end 72 has circular eye 75 for receiving actuating rod pin 71. The proximal end 72 of actuating rod 70 is pivotally mounted in slot 48A of ball member 48 (which in turn is mounted in spherical cavity 41 of head 42) by pivot pin 71, which pivotally engages eye 75 and pin hole 43. Ball member 48 and spherical cavity 41 function as a ball and socket joint allowing frame 20 and rod 70 to rotate with respect to handle 30, and additionally allowing end 72 to pivot. Preferably, end 72 is rotatably mounted in the cavity 46 to allow rotation with frame 20. If rotation of frame 20 and rod 70 is not desired, then eye 75 is simply pivotally pinned in cavity 46 of head 42. The distal end 74 of actuating rod 70 has eye 77 which is pivotally mounted to connecting members 90.

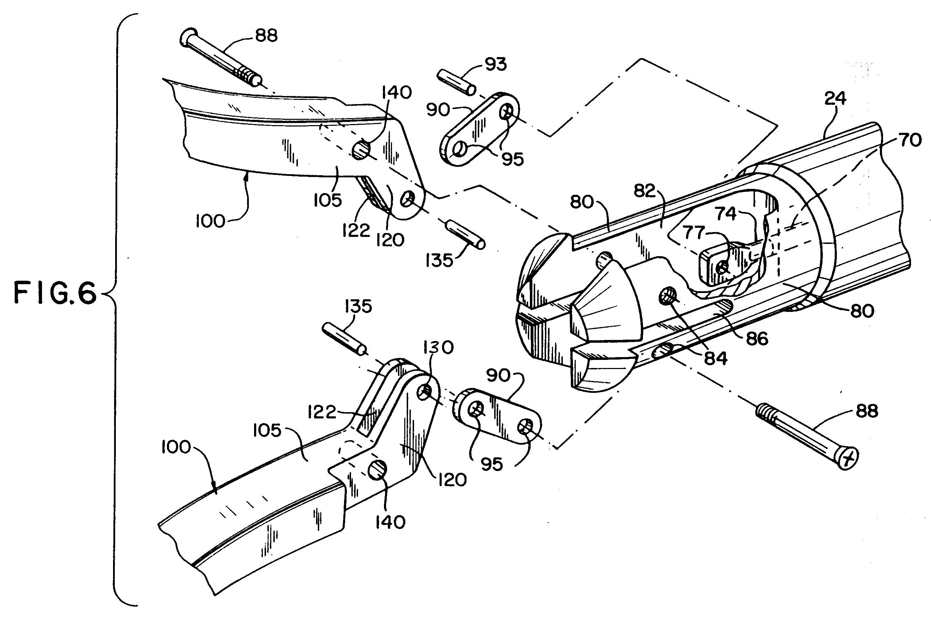

As best seen in FIG. 6, mounted to the distal end 24 of frame 20 are the jaw mounting members 80. The jaw mounting members are seen to have a semi-cylindrical shape and are disposed substantially opposite to each other and are separated by mounting slot 82. The jaw mounting members 80 have upper and lower pivot holes 84 disposed on either side of slot 86 for receiving jaw pivot pins 88. The connecting members 90 are substantially flat elongated members and have blunt, rounded ends. Centrally disposed in each end of the members 90 are the pivot pin holes 95.

The jaws 100 are elongated members having a proximal end 105 and a distal end 110. Extending from the proximal end 105 of each jaw member 100 are the angulated lever members 120. Each angulated lever member 120 has slot 122 therein for receiving a connecting member 90. It can also be seen that at the proximal end of each angulated lever member 120, there are pivot mounting holes 130 to receive pins 135. At the proximal end 105 of each jaw member 100 there are pivot mounting holes 140 for receiving jaw mounting pins 88. The jaws 100 are pivotally mounted to jaw mounting members 80 by pins 88 which are inserted through pivot holes 84 and through pivot mounting holes 140.

Members 90 are pivotally mounted on one end to jaws 100 in slots 122 by pins 135 which are inserted through pivot holes 130 and 95. Members 90 are mounted at their other end to distal end 74 of rod 70 by pin 93 through pivot holes 95 and eye 77. The above-described pins are secured using conventional techniques such as swaging, welding, screw threads, bonding with adhesives, brazing, soldering, mechanical fasteners and the like.

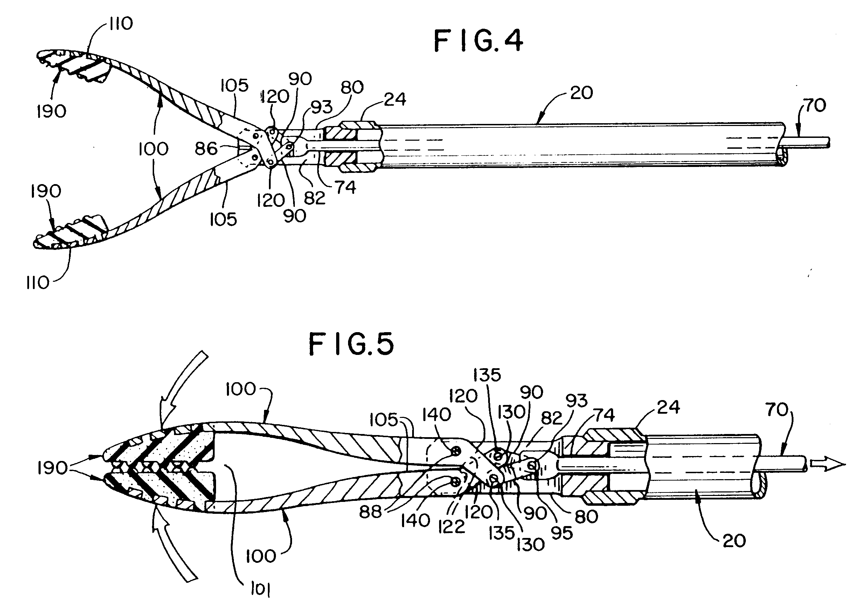

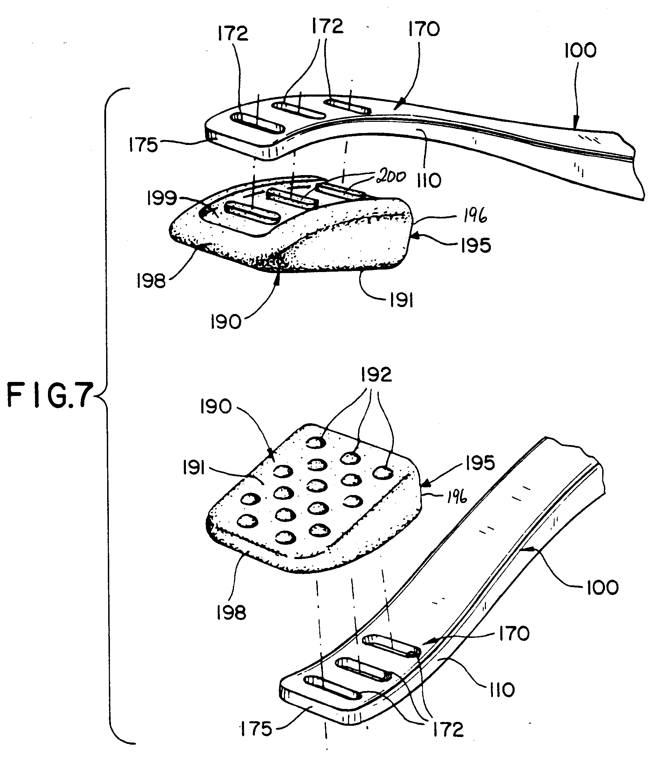

As can be seen from FIGS. 1, 2, 6 and 7, jaws 100 have a substantially rectangular cross-section at the proximal end 105 which tapers down to a substantially reduced cross-section at the distal end 110 of each jaw member 100. However, other equivalent cross-sections can be used including circular, elliptical, polyhedral and the like. Proximal to the distal end 110 of each jaw member 100 are the atraumatic mounting means 170, as seen in FIG. 7. The atraumatic mounting means 170 are seen to be curved, members extending from the reduced, distal section 110 of the jaws. Each mounting means 170 has a blunt distal end 175 and optionally at least one or more parallel slots 172 extending therethrough. As seen in Fig. 5 the 101 is seen to be contained between the jaws 100. It is particularly preferred that the jaw members 100 have a curved configuration as seen in FIG. 5 to optimize the size and configuration of the gap 101. It is believed that the presence of the gap 101 contributes to the manipulating ability of the apparatus 10.

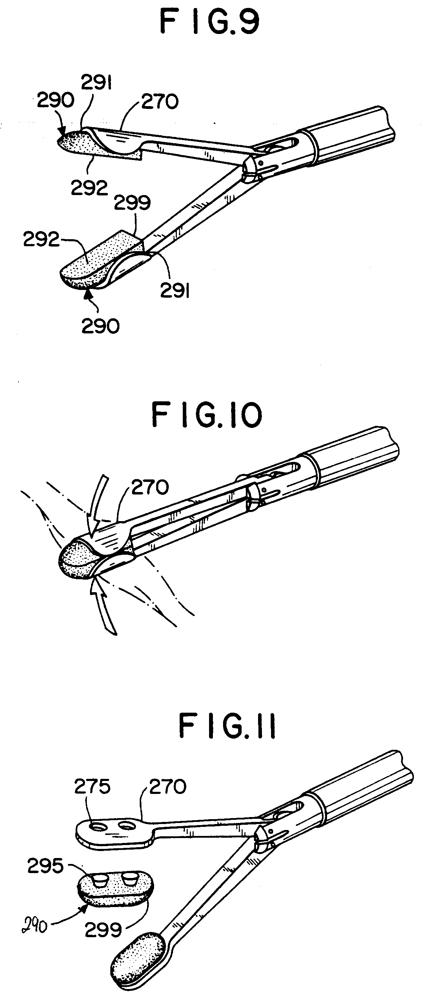

Mounted in each jaw mounting means 170 are the atraumatic means 190. The atraumatic means 190 in one embodiment as seen in FIGS 9, 10 and 11 consists of semi-cylindrical pads 290 having the curved side 291 affixed to the mounting means 270 and the flat side 292 projecting inwardly to act as a tissue contact surface. The mounting means 270 are seen to have a configuration which substantially conforms to the semi-cylindrical shape of the pads 290. As seen in the embodiment shown in FIG. 11, the curved side 291 has optional projections 295 for mounting in optional holes 275 contained in mounting means 270. The pads 290 are also seen to have proximal tissue contact surface 299.

In a preferred embodiment as seen in FIG. 7, the atraumatic means 190 consist of a wedge shaped pad 195 mounted in each mounting means 170. The pads 195 have a substantially flat, lower surface 191 having a series of optional projections 192 extending outwardly therefrom. the surface 191 may also be curved. The projections may have a variety of shapes including semi-spherical, conical, cylindrical and the like. The pads 195 will be mounted in mounting means 170 so that the flat surfaces 191 and projections 192 will contact tissue or organs. The pad 195 is seen to have a rounded blunt tip 198 and a curved upper surface 199 conforming to the shape of the mounting means 170. Upper surface 199 has a series of optional projections 200 which fit into slots 172 and are preferably frictionally engaged therein. The atraumatic pad 195 is also seen to have proximal surface 196.

Proximal surface 196 is seen to be substantially perpendicular to surface 1911 however, surface 196 may be angulated with respect to the longitudinal axis of surface 191 at an obtuse or acute angle ranging from 45 degrees to 165 degrees. Proximal surface 196 may be flat or planar. Tissue or organs grasped between jaws 100 in gap 101 will typically contact at least part of proximal surface 196.

The atraumatic means 190, in particular, the pads 195, simulate grasping by a surgeons gloved finger tips. The atraumatic means 190, such as pads 195, are characterized as sufficiently soft, and compressible, such that the pads are effective to be atraumatic to tissue and capsular organs. The atraumatic means 190 will also be sufficiently flexible, and conformal to be effectively atraumatic. By atraumatic is meant the capability to contact, grasp and maneuver tissue with minimal trauma or damage to the tissue or organs. The atraumatic means 190 will have a durometer which is the same as the durometer of human fingers (this range is widely known in the art). It is particularly preferred to manufacture the atraumatic means 190, e.g., pads 195, from polymeric foam materials. Such polymeric foam materials include such biocompatible materials such as polyethylene, polypropylene, polyurethane and the like. In addition to foam pads, the atraumatic means may be air filled plastic pads, saline filled plastic pads, gel filled plastic pads, gauze pads, cotton pads, silicone filled pads, combinations thereof and the like.

As can be seen from Figs. 1, 2, 3, 4 and 5, a counter-clockwise rotation of actuating lever 40 about the lever pin 50 causes a distal longitudinal displacement of actuating rod 70. Actuating rod 70 displaces connecting members 90 which in turn displace the jaws 100 by acting on the angulated levers 120. This causes the jaws 100 to open by pivoting about pivot pins 88. Similarly a clockwise displacement of actuating lever 40 will in a similar manner cause the actuating rod 70 to displace in a longitudinal, proximal manner causing the jaws 100 to rotate to a closed position.

The atraumatic, endoscopic apparatus 10 of the present invention is used in conventional endoscopic surgical procedures, and equivalents thereof, to manipulate tissue and body organs, in particular, capsular organs. In a conventional endoscopic surgical procedure, the patient is prepared using conventional surgical preparatory techniques including, as required, depilation of the epidermis, scrubbing, and application of aqueous iodine solutions in the area where incisions are likely to be made. Then, the patient is anesthetized using conventional anesthesiology procedures with a conventional anesthesia and the patient is connected to a ventilator an/or anesthesia machine, as required. Next, the patient's body cavity, e.g., abdominal cavity, is typically insufflated with a sterile gas such as carbon dioxide, although it is possible to operate without using insufflation. Then, using conventional endosurgical techniques, several trocar cannulas are inserted into the patient's abdominal cavity to act as pathways to and from the body cavity. Next, an endoscope is inserted through one of the trocar cannulas and the other trocar cannulas are used for the insertion of various conventional endoscopic surgical instruments including staplers, electrocautery instruments, cannulas, ligating clip appliers and the like. In order to access a particular surgical site within the body cavity, the surgeon must frequently manipulate internal organs such as the liver out of the operating field. The movements must be made in a delicate, gentle manner to minimize trauma to organs and tissue, especially the organs and tissue described herein. This can be accomplished by using the atraumatic apparatus 10 of the present invention.

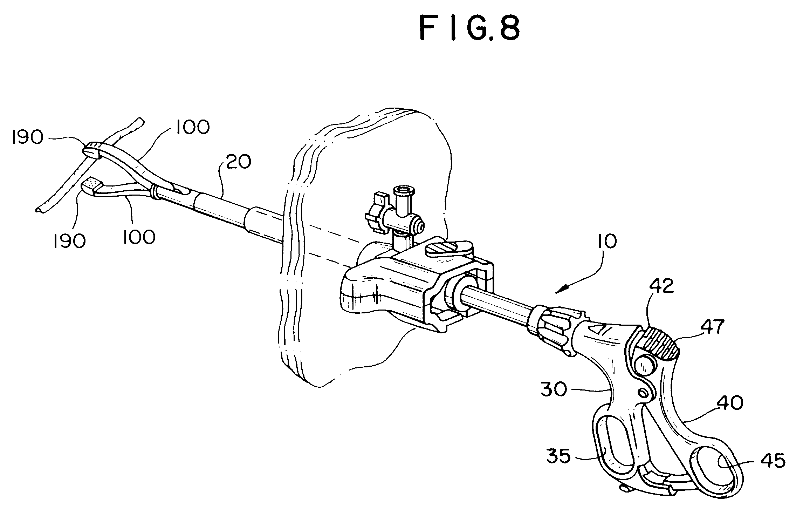

In order to use the apparatus 10, the surgeon grasps the instrument by the handle 30 with the actuating lever 40 rotated fully clockwise to a closed position so that the jaws 100 are in a closed position. Then the distal end of the apparatus 10 is inserted into a trocar cannula and displaced into the patient's body cavity. When the surgeon observes on the endoscope's visual display, typically a video monitor, that the jaws 100 of the apparatus 10 are in the body cavity, the surgeon maneuvers the jaws 100 proximate to the organ which must be moved in order to access the target surgical site, as seen in FIG. 8. Then, the surgeon rotates the actuating lever 40 in a counter clockwise manner using the thumb ring 45, thereby actuating and opening up the jaws 100. The surgeon then manipulates the open jaws 100 around a section of the organ and once again engages the actuating lever 40, this time rotating it in clockwise manner to close the jaws 100 and engage the atraumatic means 190 about the section of organ. At least one and preferably both of the surfaces 191 and 196 contact the organ and preferably tissue will be contained in gap 101. This allows the surgeon to maneuver the organ in an atraumatic manner. After displacing the organ, the surgeon manipulates the actuating means 40 to open the jaws 100 and release the organ from the atraumatic means 190. The apparatus 10 may then be withdrawn from the trocar cannula.

The atraumatic, endoscopic apparatus 10 of the present invention has many advantages for use in endoscopic surgical procedures. In particular, it is now possible for a surgeon to manipulate and move sensitive organs and tissue, such as capsular organs including the liver, spleen, and lungs, with minimal trauma, or possibly no trauma, caused to the tissue or organs. The use of the apparatus 10 having compressible atraumatic means 190 also provides the surgeon with tactile input to the hand which is holding the apparatus 10. This tactile input is an indicator of the force being applied to the organ or tissue. This tactile input is not available with conventional endoscopic instruments having hard, non-compressible surfaces. It is believed that the combination of surface 191 and proximal surface 196 results in unexpectedly improved atraumatic tissue grasping.

Although this invention has been shown and described with respect to detailed embodiments thereof, it will be understood by those skilled in the art that various changes in form and detail thereof may be made without departing from the scope of the claimed invention.

## Claims

1. An endoscopic apparatus (10) for atraumatically engaging mammalian tissue, comprising:
a frame (20) having a proximal end (22) and a distal end (24), said frame (20) having a passage (23) therethrough;
a handle means (30) mounted to the proximal end (22) of the frame (20) for holding the apparatus (10);
jaw means attached to the distal end (24) of the instrument, moveable between a fully open position and c fully closed position;
actuating means for moving the jaw means between fully extended and fully closed positions; and characterised in that it also comprises
atraumatic means (190) mounted to the jaw means effective to make the apparatus atraumatic when the jaw means are actuated, wherein said atraumatic means (190) have the same durometer as a human finger.

2. The endoscopic apparatus (10) of claim 1 wherein the actuating means comprises,
a handle lever (40) pivotally mounted to the proximal end (22) of the frame (20);
an elongated member (70) housed within said frame (20) and pivotally attached at the proximal end (22) to the handle lever (40) and,
connecting member (90) pivotally mounted at one end to the distal end (74) of the elongated member, (70) and the other end of said connecting members (90) being mounted to the jaw means.

3. The endoscopic apparatus (10) of claim 1 or claim 2 wherein the jaw means comprises a pair of pivotally mounted, opposed longitudinal members (100) having a distal end (110) and a proximal end (105), said members (100) having distal mounting means (170) for receiving the atraumatic means (190).

4. The endoscopic apparatus (10) of claim 3 wherein the longitudinal members (100) are curved.

5. The endoscopic apparatus (10) of claim 3 or claim 4 wherein the longitudinal members (100) further comprise angulated lever arms (120) projecting from the proximal end (105) of each longitudinal member (100) for pivotally mounting said connecting members (90).

6. The endoscopic apparatus (10) of any of claims 3 to 5 wherein the atraumatic means (190) comprise at least one pad member (195) mounted to each distal mounting means (170), said pad (195) having an inner surface (191) for engaging tissue, a proximal surface (196) for engaging tissue, and an outer surface (199) for mounting to the distal mounting means (170).

7. The apparatus (10) of claim 6 wherein the pad member (195) further comprises a plurality of projections (192) extending from the inner surface (191).

8. The apparatus (10) of claim 6 or claim 7 wherein the pad member (195) comprises at least one projection (200) extending from the outer surface (199) for mounting in the mounting means (170).

9. The endoscopic apparatus (10) of any of claims 3 to 8 wherein the distal mounting means (170) comprises a blunt distal end (175).

10. The endoscopic apparatus (10) of any of claims 3 to 9 further comprising a gap (101) between the longitudinal members (100) when the jaw means are fully closed.

11. The endoscopic apparatus (10) of any of claims 3 to 10 wherein the distal mounting means (170) comprises at least one slot (172) extending therethrough.

## Patentansprüche

1. Endoskopisches Gerät (10) zum atraumatischen Erfassen von Mammalia-Gewebe umfassend:
einen Schaft (20) mit einem proximalen Ende (22) Und einem distalen Ende (24) sowie einem durchgehendem Kanal (23);
einen am distalen Ende (24) des Schaftes befestigten Griff (30) zum Halten des Gerätes (10);
eine am distalen Ende (24) des Gerätes befestigte Klemmeinrichtung, die zwischen einer vollständig offenen und einer vollständig geschlossenen Position bewegbar ist;
eine Betätigungseinrichtung zum Bewegen der Klemmeinrichtung zwischen der vollständig geöffneten und der vollständig geschlossenen Position, dadurch gekennzeichnet, daß es weiterhin umfaßt:
eine an der Klemmeinrichtung befestigte atraumatische Ausstattung (190), welche das Gerät bei Betätigung der Klemmeinrichrtung atraumatisch macht, wobei die atraumatische Ausstattung (190) das gleiche Härtemaß hat, wie ein menschlicher Finger.

2. Endoskopisches Gerät (10) nach Anspruch 1, bei welchem die Betätigungseinrichtung umfaßt:
einen am proximalen Ende (22) des Schaftes (20) schwenkbar angebrachten Griffhebel (40);
ein im Schaft (20) untergebrachtes langgestrecktes Element (70), welches am proximalen Ende (22) schwenkbar mit dem Griffhebel (40) verbunden ist und
Verbindungselemente (90), welche an ihrem einem Ende schwenkbar mit dem distalen Ende (74) des langgestreckten Elementes (70) und an ihrem anderen Ende schwenkbar mit der Klemmeinrichtung verbunden sind.

3. Endoskopisches Gerät (10) nach Anspruch 1 oder 2, bei welchem die Klemmeinrichtung ein Paar schwenkbar befestigter, einander gegenüber angeordneter Längselemente (100) mit einem distalen Ende (110) und einem proximalen Ende (105), wobei diese Elemente (100) distale Befestigungseinrichtungen (170) zur Aufnahme der atraumatischen Ausstattung (190) aufweisen.

4. Endoskopisches Gerät (10) nach Anspruch 3, bei welchem die Längselemente (100) gekrümmt sind.

5. Endoskopisches Gerät (10) nach Anspruch 3 oder 4, bei welchem die beiden Längselemente (100) weiterhin abgewinkelte Hebelarme (120) aufweisen, welche zur schwenkbaren Anbringung der Verbindungselemente (90) am proximalen Ende (105) eines jeden Längselementes (100) herausragen.

6. Endoskopisches Gerät (10) nach Anspruch 3 bis 5, bei welchem die atraumatische Ausstattung (190) zumindest ein an jeder der distalen Befestigungseinrichtungen (170) befestigtes Polster (195) umfaßt, wobei das Polster (195) eine Innenfläche (191) zur Anlage am Gewebe, eine proximale Fläche (196) zur Anlage am Gewebe und eine Außenfläche (199) zur Befestigung an der Befestigungseinrichtung (170) aufweist.

7. Endoskopisches Gerät (10) nach Anspruch 6, bei welchem das Polster (195) weiterhin eine Vielzahl von Vorsprüngen (192) aufweist, welche an der Innenfläche (191) aufragen.

8. Endoskopisches Gerät (10) nach Anspruch 6 oder 7, bei welchem das Polster (195) an der Außenfläche (199) mindestens einen Vorsprung (200) zur Befestigung an der Befestigungseinrichtung (170) aufweist.

9. Endoskopisches Gerät (10) nach einem der Ansprüche 3 bis 8, bei welchem die distale Befestigungseinrichtung (170) ein abgerundetes distales Ende (175) aufweist.

10. Endoskopisches Gerät (10) nach einem der Ansprüche 3 bis 9, welches weiterhin bei vollständig geschlossener Klemmeinrichtung einen Spalt (101) zwischen den Längselementen (100) aufweist.

11. Endoskopisches Gerät (10) nach einem der Ansprüche 3 bis 10, bei welchem die distale Befestigungseinrichtung (170) mindestens einen, sich in dieser erstreckenden Schlitz (172) aufweist.

## Revendications

1. Instrument endoscopique (10) pour venir en prise, sans trauma, avec un tissu de mammifère, comportant:
un cadre tubulaire (20) présentant une extrémité arrière (22) et une extrémité avant (24), ledit cadre tubulaire (20) présentant un passage traversant (23);
des moyens de maniement (30) montés a l'extrémité avant (24) du cadre tubulaire (20) pour tenir te dispositif (10);
des moyens formant mâchoires, attachés a l'extrémité avant (24) de l'instrument et pouvant se déplacer entre une position ouverte et une position entièrement fermée;
des moyens de manoeuvre pour faire passer les moyens formant mâchoire entre une position entièrement en extension et une position entièrement fermée, et caractérisé par le fait qu'il comporte également
des moyens (190) atraumatiques, montés sur les moyens formant mâchoire, efficaces pour faire en sorte que l'instrument évite le trauma lorsqu'on manoeuvre les moyens formant mâchoires, dans lequel, lesdits moyens (190) atraumatiques ont la même valeur au duromètre qu'un doigt humain.

2. Instrument endoscopique (10) de la revendication 1 dans lequel les moyens de manoeuvre comportent
un levier de maniement (40) monté avec liberté de pivotement, a l'extrémité arrière (22) du cadre tubulaire (20);
un élément allongé (70) logé a l'intérieur dudit cadre (20) et attaché, avec liberté de pivotement, a l'extrémité arrière (22) du levier de maniement (40); et
des éléments de connexion (90) montés, avec liberté de pivotement, a une première extrémité, a l'extrémité avant (74) de l'élément allongé (70), et l'autre extrémité desdits éléments de connexion (90) étant montée sur les moyens formant mâchoire.

3. Le dispositif endoscopique (10) de la revendication 1 ou de la revendication 2, dans lequel les moyens formant mâchoire comportent une paire d'éléments longitudinaux, (100), opposés, montés avec liberté de pivotement, ayant une extrémité avant (110) et une extrémité arrière (105), lesdits éléments (100) ayant des moyens de montage avant (170) pour recevoir les moyens (190) atraumatiques.

4. L'instrument endoscopique (10) de la revendication 3, dans lequel les éléments longitudinaux (100) sont incurvés.

5. L'instrument endoscopique (10) de la revendication 3, dans lequel les éléments longitudinaux (100) comportent en outre des bras de levier en angle (120) débordant de l'extrémité arrière (105) de chaque élément longitudinal (100) pour se monter, avec liberté de pivotement, sur lesdits éléments de connexion (90).

6. L'instrument endoscopique (10) de l'une des revendications 3 à 5, dans lequel les moyens (190) atraumatiques comportent au moins un élément formant bourrelet (195) monté sur chaque moyen de montage avant (170); ledit bourrelet (195) ayant une surface intérieure (191) pour venir en prise avec un tissu, une surface arrière (196) pour venir en prise avec un tissu et une surface extérieure (199) pour montage sur les moyens de montage avant (170).

7. Le dispositif (10) de la revendication 6, dans lequel l'élément formant bourrelet (195) comporte en outre une pluralité de bossages (192) s'étendant depuis la surface intérieure (191).

8. Le dispositif (8) de la revendication 6 ou de la revendication 7, dans lequel l'élément formant bourrelet (195) comporte au moins une nervure (200) s'étendant depuis la surface extérieure (199) pour montage sur les moyens de montage (170).

9. L'instrument endoscopique (10) de l'une quelconque des revendications 3 à 8, dans lequel les moyens de montage avant (170) comportent une extrémité avant émoussée (175).

10. L'instrument endoscopique (10) de l'une quelconque des revendications 3 à 9, comportant en outre un jeu (10) entre les éléments longitudinaux (100) lorsque les moyens formant mâchoire sont entièrement fermés.

11. L'instrument endoscopique (10) de l'une quelconque des revendications 3 à 10, dans lequel les moyens de montage avant (170) comportent au moins une fente traversante (172).
